# EUROPEAN PATENT APPLICATION

(11) **EP 1 498 123 A1**
(43) Date of publication of application: **19.01.2005**
(21) Application number: 03291797.3
(22) Date of filing: 18.07.2003
(51) Int. Cl.: A61K 31/397, A61K 31/135, A61K 47/44

(54) **Emulsifying systems containing azetidine derivatives**

(71) Applicant: Aventis Pharma S.A., 92160 Antony (FR)
(72) Inventor: Cote, Sophie, 92160 Antony (FR); Peracchia, Maria-Teresa, 75005 Paris (FR); Bobineau, Valérie, 91370 Verrieres le Buisson (FR)
(74) Representative: Le Pennec, Magali

(57) **Abstract**

Emulsifying systems containing azetidine derivatives. Emulsifying systems containing azetidine derivatives. The present invention relates to novel formulations of azetidine derivatives for oral administration.

## Description

The present invention relates to stable formulations of azetidine derivatives.

The azetidine derivatives used in the pharmaceutical compositions according to the invention may be designated by the general formula (Ia) or (Ib) below: 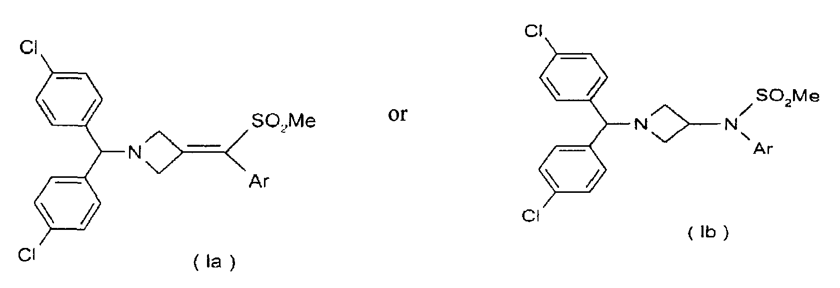 in which Ar is an aromatic or heteroaromatic group optionally substituted with one or more (C1-C4)alkyl, halogen, NO₂, CN, (C1-C4)alkoxy or OH groups.

In the definition of the azetidine derivatives above, aromatic group is understood to mean in particular a phenyl or naphthyl group, heteroaromatic group a pyridyl, furyl, thienyl, thiazolyl, imidazolyl or oxazolyl group, and halogen fluorine, chlorine, bromine or iodine.

Compound (Ic) below, is a specific example of azetidine of general formula (Ia):

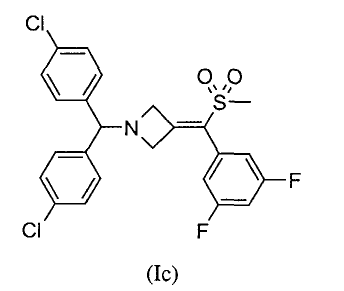

In patent applications WO 00/15609, WO 01/64633, WO 0064634 and WO 99/01451, there have been described azetidine derivatives of general formula (Ia) or (Ib) and their applications. In particular, these azetidine derivatives are particularly advantageous for their high affinity for cannabinoid receptors and in particular CB1-type receptors.

Unfortunately, azetidine derivatives are products that are only very slightly water-soluble. Up until now, it was envisaged to administer the azetidine derivatives of general formula (Ia) or (Ib), in particular by the oral route, in the form of tablets in formulations comprising, inter alia, cellulose, lactose and other excipients. However, such formulations are not always sufficiently well suited to these sparingly. water-soluble products because of an excessively low bioavailability.

Numerous documents describe systems suitable for solubilizing and/or enhancing the bioavailability of hydrophobic active ingredients. However, the systems tested have so far proved ineffective for the preparation of pharmaceutical compositions containing azetidine derivatives defined above which are stable and bioavailable and in which the azetidine derivative is solubilized at an effective concentration.

In particular, J. Pharm Sciences, 89(8), 967 (2000) and Pharmaceutical Technology Europe, p. 20, September 2000 mention the formulation of active ingredients which are sparingly soluble in water, in medium-chain triglycerides. However, the trials carried out with formulations based on Miglyol® have given insufficient results from the point of view of their bioavailability.

Moreover, international application WO 95/24893 describes compositions comprising digestible oil, a lipophilic surfactant and a hydrophilic surfactant, which are intended for the formulation of hydrophobic active ingredients and for the enhancement of their bioavailability. International patent application PCT/FR02/04514 explains that the above azetidine derivatives are too weakly bioavailable in this type of formulation. In particular, the formulation of such azetidine derivatives in a Miglyol®/Capryol®/Cremophor® system is insufficient in vivo from the pharmacokinetic point of view.

It has now been found, and that is what constitutes the subject of the present invention, that it is possible to prepare chemically and physically stable pharmaceutical compositions comprising a derivative of general formula (Ia) or (Ib), optionally in combination with another active ingredient capable of potentiating the effects of the azetidine derivative of general formula (Ia) or (Ib), in a system comprising:
(i) a phospholipid solubilized in a liquid solvent, or
(ii) a combination of Miglyol, Capryol and Cremophor. Indeed, it has been found that formulations of compound (Ia) or (Ib) with a phospholipid allow obtaining unexpected very good *in vivo* pharmacological kinetic profile, as concerns AUC, Cmax and Cmax variability. On the same manner, and contrarily to what was said earlier in PCT/FR02/04514, it has been observed that formulations of compound (Ia) or (Ib) with microemulsions obtained via combination of Miglyol, Capryol and Cremophor allow obtaining very good *in vivo* pharmacological kinetic profile for one dog among the three dogs evaluated, as concerns AUC, and Cmax. The observed vomiting could be one of the reasons of the disappointing results in the other two dogs.

A preferred formulation contains compound (Ic).

The pharmaceutical formulation preferably contains up to 200 mg azetidine derivative per g.

The pharmaceutical composition may further comprise an additional additive chosen from stabilizing agents, preservatives, agents which make it possible to adjust the viscosity, or agents which can modify, for example, the organoleptic properties.

For certain treatments such as, for example, obesity, it may be advantageous to administer the azetidine derivatives of general formula (Ia) or (Ib) at the same time as sibutramine, which causes a synergistic effect in the reduction of food consumption.

Sibutramine and its effects have been described in the references below: WO 90/061110; D. H. RYAN et al., Obesity Research, 3 (4), 553 (1995); H. C. JACKSON et al., British Journal of Pharmacology, 121, 1758 (1997); G. FANGHANEL et al., Inter. J. Obes., 24 (2), 144 (2000); G. A. BRAY et al., Obes. Res., 7(2), 189 (1999).

Moreover, for other treatments such as schizophrenia or the treatment of neurological disorders such as Parkinson's disease, it may be advantageous to administer the azetidine derivatives of general formula (Ia) or (Ib) at the same time as one or more agents, which activate dopaminergic neurotransmission in the brain. These combinations make it possible to potentiate the effects of a dopaminergic monotherapy (levodopa, dopaminergic agonists, and inhibitors of enzymes), and make it possible to reduce side effects, in particular dyskinesia.

Among the dopaminergic agonists, the following products may be mentioned in particular: bromocriptine (Novartis), cabergoline (Pharmacia Corp.) adrogolide (Abbott Laboratories), BAM-1110 (Maruko Seiyaku Co Ltd), Duodopa® (Neopharma), L-dopa, dopadose (Neopharma), CHF1512 (Chiesi), NeuroCell-PD (Diacrin Inc), PNU-95666 (Pharmacia & Upjohn) ropinirole (GlaxoSmithKline Beecham), pramipexole (Boehringer Ingelheim) rotigotine (Discovery Therapeutics, Lohmann Therapie System), spheramine (Titan Pharmaceuticals), TV1203 (Teva pharmaceutical), uridine (Polifarma).

It is understood that the compositions comprising, in addition, an active ingredient other than the azetidine derivative of general formula (Ia) or (Ib) and capable of potentiating the effects thereof may contain a product as defined in the paragraphs above and that said compositions fall within the scope of the present invention.

The active ingredient derived from azetidine is preferably present in an amount of 0.01 to 70% by weight of the total composition.

According to another aspect, the invention is about a process for preparing a composition comprising an azétidine according to its first aspect, wherein there is prepared, where appropriate, the mixture of principal excipients, after heating, if necessary, in the case of the solid or semisolid excipients, and then, if necessary, the mixture with the additional additives, and then the azetidine derivative (Ia) or (Ib), where appropriate, the active ingredient capable of potentiating the effects of the azetidine derivative of general formula (Ia) or (Ib), defined in claim 1 are added and stirring is maintained in order to obtain a homogeneous mixture.

According to a further aspect, the invention is about a presentation kit containing a composition as defined above, and a composition comprising an active ingredient capable of potentiating the effects of the azetidine derivative (Ia) or (Ib).

The active ingredient of the presentation kit capable of potentiating the effects of the azetidine derivative is preferably sibutramine.

According to a last aspect, the invention is about a presentation kit containing a composition according ot its first aspect, and a composition comprising an agent which activates dopaminergic neurotransmission in the brain.

In the first preclinical studies performed in rats, the oral administration of an aqueous suspension of a drug substance of formula (I) in 0.5% methylcellulose / 0.2% tween 80 (dose at 10 mg/kg) led to a very low bioavailability (3%). A first formulation approach has been to use a solution of 25 mg/mL (Ic) in Miglyol 812N, chosen because of the higher drug substance solubility in oily components (35.9 mg/mL in Miglyol 812). Furthermore, this excipient (medium chain triglyceride) is known for its digestibility and regulatory acceptability. This formulation has been used for further preclinical studies, leading to an increased bioavailability of the drug substance of formula (Ic) in rats (13 and 37% with doses at 1mg/kg and 10 mg/kg respectively). However, in the First in Man study, an important food effect and interindividual variability were observed: the Maximum Tolerated Dose was around 100 mg in fasted conditions, with an interindividual variability of 50 %, whereas in fed conditions the Maximum Tolerated Dose was divided by 10 with a decrease of the interindividual variability to 30%.

Based on all these results, the requirements for the development of a new formulation were the following:
1. to develop a formulation with a lower drug concentration (10 mg/g instead of 25 mg/g)
2. to increase the bioavailability
3. to reduce the interindividual variability
4. to reduce the food effect (fed/fasted conditions)

The development of non-standard formulations arised with use of excipients able to increase the solubilization of the active.

Thus, other lipidic excipients have been investigated for further formulations, with the aim of enhancing the drug solubilisation/absorption steps. Efforts were focused on "Lipid-based formulations", able to form *in situ* a homogeneous and fine emulsion or microemulsion or micellar solution, due to the surfactant properties of the excipients.

Indeed, Miglyol exhibits emulsifying properties, but it forms, in contact with an aqueous medium, an heterogeneous and rough emulsion (large oily drops macroscopically visible), that could explain the in vivo results.

"Lipid-based formulations" range from pure oils to blends, which contain important amounts of surfactants and cosolvents (higher polarity). First the solubility compound (Ic) in an exhaustive series of lipids and other pharmaceutical cosolvents was determined.

Three categories of excipients were identified and chosen for compound (Ic)formulation:
- Amphiphilic excipients to administer as solutions (Phosal 50PG, Labrasol), able to self-emulsify once in contact with the physiological medium (droplet size 1-10 µm).
- Amphiphilic excipients to administer as semi-solid matrices (Gelucire 44/14, Vitamin E TPGS) for drug solubilization by micellarization (droplet size <20 nm).
- Mixture of lipidic excipient (Miglyol 812N) blended with a surfactant (Cremophor RH40or EL) and a cosolvent (Capryol 90): this mixture is able to self micro emulsify in-situ with gastro-intestinal fluids (droplet size < 20 nm).

All the chemical compositions of the selected excipient, as well as their physicochemical properties and other main characteristics, are described further on.

In the present work, all the identified formulation prototypes were evaluated in terms of in vitro behaviour in physiological conditions, after dilution and incubation with simulated gastro-intestinal fluids. The following parameters were investigated: microscopic/macroscopic aspect of the obtained particulate dispersion; determination of the solubilized fraction of the drug before and after incubation of the formulation (in presence or not of an intestinal enzyme); evaluation of the colloidal stability of the dispersion after incubation.

The obtained results allowed a first screening for identifying the formulation prototypes potentially interesting for in vivo studies.
Figure 1: Compound (Ic) aqueous solution: effect of the filter size on compound (Ic) fraction recovered

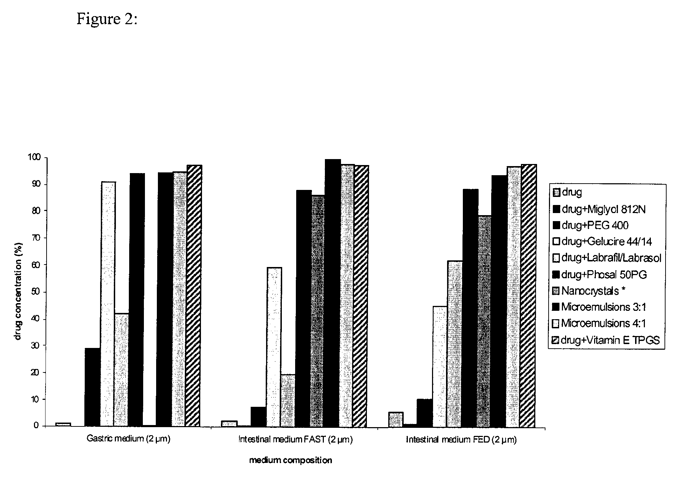

Figure 2: - Compound (Ic) lipidic formulations: effect of the medium on compound (Ic) concentration after 2h agitation at 37°C and filtration (2 µm)

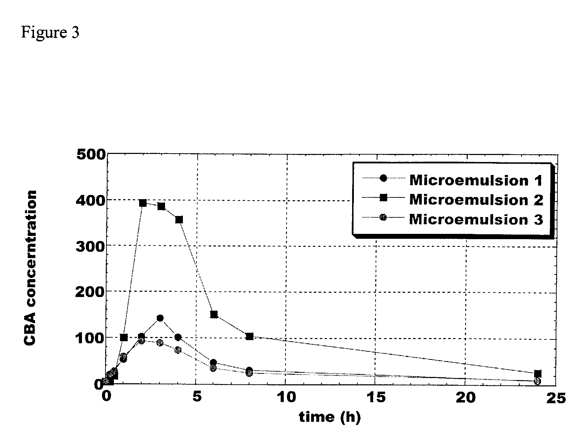

Figure 3: - Compound (Ic) PK profile in microemulsion formulation in 3 different Beagle dogs.

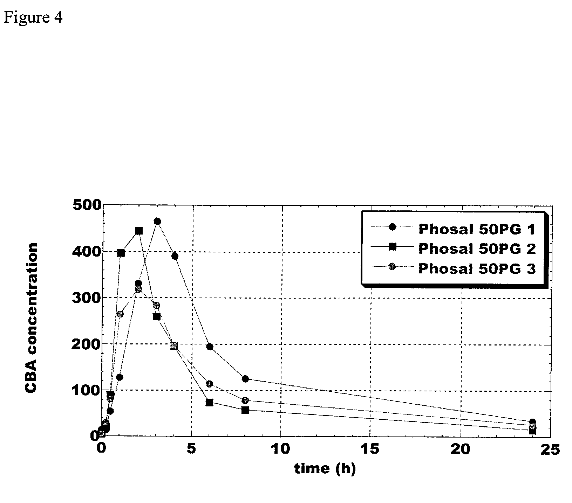

Figure 4: - Compound (Ic) PK profile in Phosal 50PG formulation in 3 different Beagle dogs.

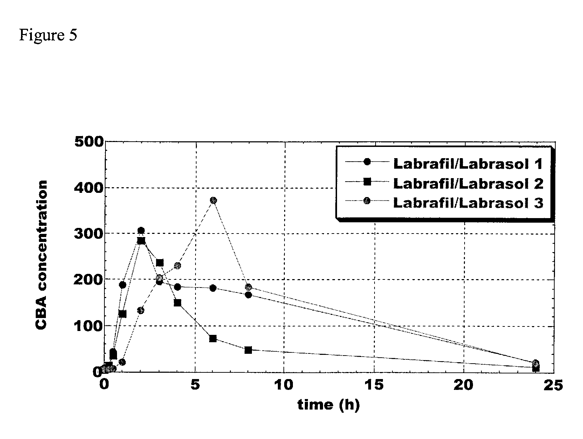

Figure 5: - Compound (Ic) PK profile in Labrafil/Labrasol formulation in 3 different Beagle dogs.

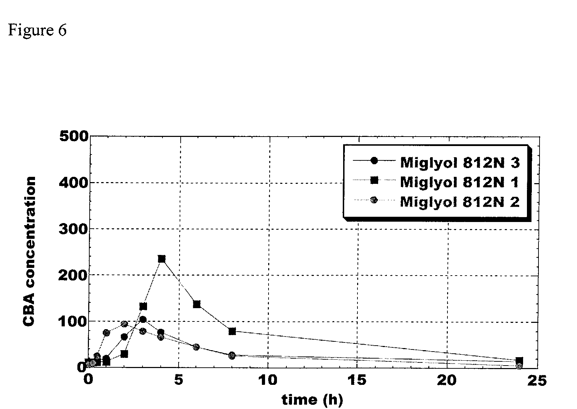

Figure 6: - Compound (Ic) PK profile in Miglyol 812N formulation in 3 different Beagle dogs.

### formulation of the prototypes

Preamble: Description of the excipients
(i) Vitamin E TPGS (Eastman Chemicals):
   Vitamin E TPGS (d-α-tocopheryl polyethylene glycol 1000 succinate) is a water-soluble derivative of natural-source vitamin E, of non-animal origin.
(ii) Phosal 50PG (Aventis Nattermann)
   Phosal 50PG is a phosphatidylcholine concentrate with at least 50% PC and propylene glycol.
   Composition: Phosphatidylcholine app. 56.8%
   Propylene glycol: app. 38%
   Sunflower mono/diglycerides: app. 3%
   Soybean fatty acids: app. 2%
   Ascorbyl palmitate: app. 0.2%; Ethanol: ad 100%
   It is synthesized starting from soya lecithin, purified into Phospholipon and then solubilized in a liquid carrier system.
   Nattermann Phospholipid GmbH sells also other phospholipids that are solubilised in varying forms. For instance, Phosal ® 53MCT, that is a form consisting of phosphatidylcholine solubilised in a carrier system comprising caprylic/capric triglycerides, alcohol, glyceryl stearate, oleic acid and ascorbyl palmitate. The phosphatidylcholine content is about 56 ± 3 % w/w.
(iii) Labrasol (Gattefosse)
   Labrasol® (Caprylocaproyl Macrogol-8 Glycerides) is a saturated polyglycolized glyceride consisting of mono-, di- and triglycerides and of mono- and di-fatty acids of polyethylene glycol (PEG)
   This amphiphilic oil obtained from vegetable and petrochimic origin is soluble in water.
(iv) Labrafil (Gattefosse)
   Labrafil® M 1944 CS (Oleic Macrogol-6 Glyceride), an amphiphilic oil dispersible in water (HLB 4), derived from selected high purity vegetable oils. This excipient allows increasing the lipophilic character of the formulation prototype with the aim to improve the solubilization of the active in gastrointestinal fluids by formation of fine dispersion. In addition, this material miscible with cholesterol and phospholipids, could go through the membranes by a non active mecanism (passive diffusion).
(v) Gelucire (Gattefosse)
   Gelucire® 44/14 (Lauroyl Macrogol-32 Glycerides) is a saturated polyglycolized glyceride consisting of mono-, di- and triglycerides and of mono- and di-fatty acids of polyethylene glycol (PEG).
   Gelucire® 44/14 is obtained from the reaction of hydrogenated palm kernel oil with PEG 1500.
(vi) Miglyol 812 is described as a fixed oil extracted from the hard, dried fraction of the endosperm of *Coco nucifera* L.by hydrolysis, fractionation of the fatty acids obtained and re-esterification. It consists of a mixture of exclusively short and medium chain triglycerides of fatty acids, of which not less than 95 % are the saturated acids octanoic (caprylic) acid and decanoic (capric) acid.
   It is a colourless to slightly yellowish oily liquid, which is practically odourless and tasteless.
(vii) Cremophor RH40 is a Polyoxyl 40 hydrogenated castor oil. This material is obtained by reacting ethylene oxide with hydrogenated castor oil. It occurs as a white semisolid paste that liquefies at 30°C. It has a very faint characteristic odour and a slight taste in aqueous solution.
(viii) Cremophor EL is Polyoxyl 35 castor oil (Polyoxyethyleneglycerol triricinolineate, glycerol-polyethyleneglycol ricinoleate)
   This material is obtained by reacting ethylene oxide with castor oil (German Pharmacopeia quality). Cremophor EL is a pale yellow, oily liquid (viscosity at 25°C:700-850 cP) that is clear at T>26°C. It has a slight but characteristic odour and can completely liquefied by heating to 26°C.
(ix) Capryol 90 is Propylene Glycol Monocaprylate
   This material is obtained from vegetable and petrochemical origin is insoluble in water.
   Liquid.

### Solubility data

The first step was to determine the solubility of compound(Ic) in an exhaustive series of lipids and other pharmaceutical cosolvents including vegetable oils, lipidic components, surfactants, hydrophilic components and phospholipids. The protocol of the solubility measurement is reported in the annex.

**Table 1 -**

| Solubility data | | |
|---|---|---|
| Commercial name | Chemical description | solubility (mg/mL) |
| Vegetable oils | | |
| Soyabean oil | | 31.8 |
| Peanut oil | | 7.2 |

| Lipidic components | | |
|---|---|---|
| Imwitor 988 | Glyceryl mono-dicaprylate | 41.2 |
| Imwitor 742 | Glyceryl mono-dicaprylate/caprate | 31.2 |
| Miglyol 812 | caprylic/capric triglyceride | 35.9 |
| Labrafil M1944 CS | oleyl macrogol-6-glycerides | 30.3 |

| Surfactants | | |
|---|---|---|
| Tween 80 | POE monooleate | 50.6 |
| Cremophor RH40 | POE hydrogenated castor oil | 80.6 |
| Span 20 | Sorbitan monolaurate | 31.6 |
| Span 85 | Sorbitan trioleate | 92.0 |

| Hydrophilic components | | |
|---|---|---|
| PEG 400 | polyethylene glycol 400 | 78.1 |

| Others | | |
|---|---|---|
| Ethanol | | 5.8 |
| HP β Cyclodextrin | | 0.05 |
| Transcutol | Diethylene glycol monoethyl ether | 89.8 |

**Table 2 -**

| Complementary solubility data | | |
|---|---|---|
| Commercial name | Chemical description | solubility (mg/g) |
| Vegetable oils | | |
| Coconut oil | | 18.6 |
| Castor oil | | 16.1 |
| Safflower oil | | 14.8 |
| Sesame oil | | 10.9 |
| Olive oil | | 10.9 |

| Lipidic components | | |
|---|---|---|
| Crossential 094 | Oleic acid | 19.6 |
| Crossential LN 75 | Linolenic acid | 45.4 |
| Edenor C8 98-100 | Caprylic acid | 75.5 |
| Myvacet 9-45 | Distilled acetylated monoglycerides | 54.8 |
| Neobee M 20 | PG dicaprylate/dicaprate | 47.7 |
| Lauroglycol | PG monolaurate | 28.5 |
| Capryol 90 | Polyethyleneglycol monocaprylate | 56.6 |

| Surfactants | | |
|---|---|---|
| Myrj 45 | POE stearate | 86.9 |
| Brij 96 | POE oleyl ether | 85.7 |
| Labrasol | Caprylocaproyl macrogol-8 glycerides | 111.8 |

| Hydrophilic components | | |
|---|---|---|
| PEG 1500 | polyethylene glycol 1500 | < 10 |
| Gelucire 44/14 | lauroyl macrogol-32 glycerides | 90.1 |
| Gelucire 50/13 | stearoyl macrogol-32 glycerides | 78.5 |

| Phospholipids | | |
|---|---|---|
| Phospholipon 90 | Phosphatidylcholine 93 % | 40 |
| Phosal 40 MD | Phosphatidylcholine 38 ± 1.5% in glyceryl stearate, soya oil and linoleic acid ethylester | 12.5 |
| Phosal 75 SA | Phosphatidylcholine 75 ± 3% in alcohol, safflower oil, glyceryl stearate, coconut oil and ascorbyl palmitate | < 10 |
| Phosal 50 PG | Phosphatidylcholine ≥ 50% in propylene glycol | 11.7 |
| Phosal 53 MCT (unstable excipient, not recommended, see next page) | Phosphatidylcholine 56 ± 3% in caprylic/capric triglycerides, alcohol, glyceryl stearate, oleic acid and ascorbyl palmitate | 25.8 |

After determination of the solubilities, our objective was to select a few excipients taking into account the solubility of the active, their registrability and their ability to increase the bioavailability of a drug substance (by solubilisation improvement or absorption enhancement).

Concerning the third criterium, the excipients were then selected based on:
1. their amphiphilic character (HLB>10) (Labrasol, Gelucire 44/14, Phosal 50PG, Vitamin E TPGS) able to solubilize a lipophilic active and to be dispersed or dissolved in gastrointestinal fluids
2. their ability in mixture to form a microemulsion in situ after dilution with gastrointestinal fluids by the good combination of an oil, a hydrophilic surfactant (HLB>10) and a lipophilic co-surfactant (HLB<10) (Miglyol 812 /Cremophor RH40 / Capryol 90)

Concerning Phosal 53MCT, a main issue on the physical stability of the excipient led to choose Phosal 50PG as alternative. Indeed, the observed phase separation of the excipient concerned not only the batch stocked at Aventis, but also the batches stocked at Nattermann. Phosal 50PG exhibited a very good physical stability.

The main features of the selected excipients are described in the table below:

**Table 3 -**

| Main features of the selected excipients | | | |
|---|---|---|---|
| Commercial Name | Chemical description | FEATURES | Solubility |
| (Supplier) | | | (mg/g) |
| Excipients able to form fine emulsion or micellar | | solution in contact with physiological fluids | |
| Labrasol® (Gattefosse) | Caprylocaproyl macrogol-8 glycerides | Liquid / HLB 13 DMF / European monograph | 111.8 |
| Labrafil M 1944 CS | Oleic | Liquid / HLB 4 | 30.3 |
| (Gattefosse) | Macrogol-6 glycerides | DMF / European monograph | |
| Phosal® 50 PG (Natterman) | Phosphatidylcholine ≥ Viscous liquid / Amphiphilic 50% in propylene glycol properties GRAS status | | 11.7 |
| Gelucire® 44/14 (Gattefosse) | lauroyl macrogol-32 glycerides | Waxy solid / Melting point 44°C / HLB 14 GRAS status / European monograph | 90.1 |
| VitaminE TPGS (Eastman) | Vit E d-α-tocopheryl poly ethylene glycol 1000 succinate | Waxy solid / Melting point 38°C / HLB 13 GRAS status/ USP monograph | 100 |
| Mixture of excipients able to self microemulsify in contact with physiological | | | fluids |
| Miglyol 812 (Condea) | Caprylic / capric triglycerides | Oily phase / Fatty acids : C₈ and C₁₀ GRAS status / European monograph | 37.4 |
| Capryol® 90 (Gattefosse) | Polyethyleneglycol monocaprylate | Cosurfactant / Liquid / HLB 6 Food additive status | 56.6 |
| Cremophor RH 40 (BASF) | Polyoxyl 40 hydrogenated castor oil | Surfactant / semisolid / liquid at 30°C HLB 14-16 FDA inactive ingredients / USP Monograph | 80.6 |

### DESCRIPTION OF THE PROTOTYPES

### Oily solutions and semi-solid matrices (Binary and Ternary mixtures)

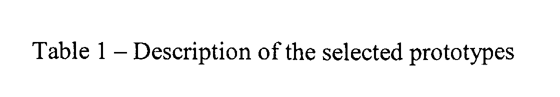

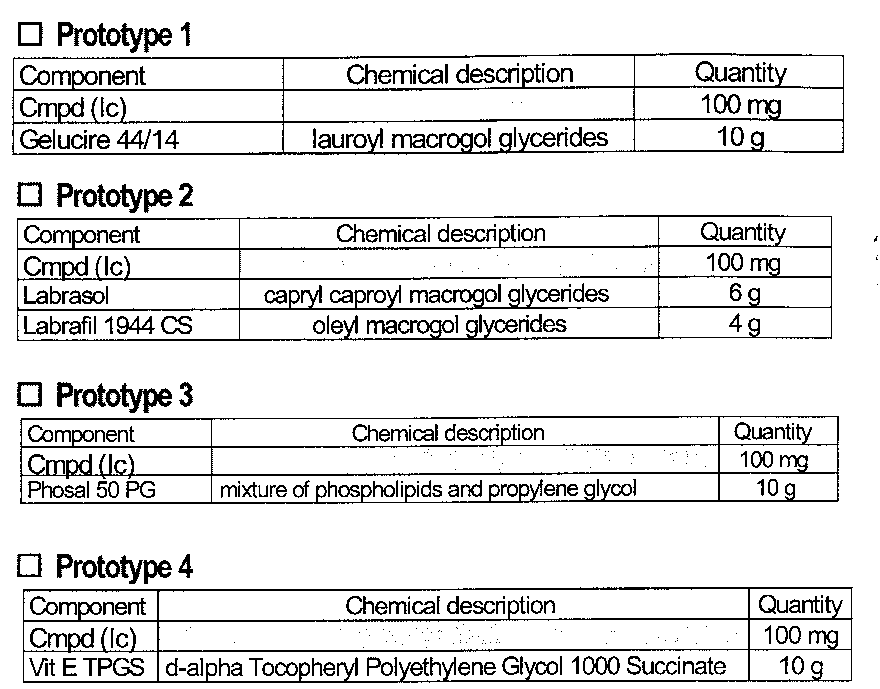

For the Labrasol prototype, the maximum amount of Labrasol to include in the prototype was 60% (w/w) because, at higher amount, a risk of incompability with the gelatin of the capsule shell was emphasized. Formulations with higher content of Labrasol could be used with capsules not made of gelatine. In order to complete the bulk composition of this formulation, it was decided to use Labrafil M 1944 CS, a lipophilic component (HLB 4), at 40% (w/w).

### Self Micro Emulsifying System : Oil/surfactant/co-surfactant mixture (pseudo-ternary mixture)

Any formulation containing an amphiphilic surfactant/cosurfactant couple leads to the formation of several micellar states. Our aim was to develop formulation prototypes able to form spontaneously a microemulsion with physiological fluids.

Microemulsions can be defined as transparent, isotropic, thermodynamically stable liquids. As a consequence, microemulsions can be dilute indefinitely. The transparency is the consequence of their microstructure which consists of micro-droplets of size < 100 nm.

Their main properties of pharmaceutical interest are : high drug solubilizing power ; dilution capacity, leaving the molecule in micellar solution in situ; and dispersion capacity with a droplet size allowing easier absorption.

### Choice of excipients

Based on the litterature and on solubility results obtained with excipients described for microemulsion formulation (see paragraph **Erreur ! Source du renvoi introuvable.),** the following components were selected, with the aim to develop one microemulsion prototype:
- oily phase : Miglyol 812
- surfactant : Cremophor RH 40
- co surfactant : Capryol 90
- aqueous phase : physiological fluids

### Pseudoternary diagram

The design of this diagram allows determining the excipients ratio able to give the region of the microemulsion. Microemulsions being quaternary systems, their graphic representation requires a tridimensional representation. However, in order to simplify the representation, a pseudo-ternary diagram is used.

The micro emulsion is assumed to be a pseudo-ternary mixture of:
1. Water phase
2. Oily phase (Miglyol 812N)
3. Surfactant / Co surfactant (Cremophor RH 40 / Capryol 90)

Protocol:
First, the ratio S/CoS is defined: 4 different ratios were tested (1:1; 2:1, 3:1 and 4:1). For each ratio, the pseudo-ternary diagram is designed by setting:
   - The percentage of the oily phase (20%, 40%, 60% and 80%)
   - The percentage of the couple S/CoS (100% less the oil percentage)
Then, the water phase is added drop by drop. The percentage of each "component" is thus modified after each water addition.

The modification of the visual aspect from turbid to translucid and inversely, shows the borders of the microemulsion region. In addition, a measurement of the droplet size before and after infinite dilution (Coulter Nanosizer N4+) allowed confirming the formation of a microemulsion.

The pseudoternary diagram was obtained without the active and performed again with the active.

Results:
• A microemulsion area was observed with the S/CoS ratio of 3:1 and 4:1. (see diagrams). In this area, the droplet size was around 25 nm.
The micro emulsion area was obtained for a low initial percentage of oil phase (20%) whatever the S/CoS ratio (3:1 and 4:1) and with a high quantity of water (from 55 % to 86 %).
The initial composition of the self microemulsifying systems were:

| | Ratio 3:1 | Ratio 4:1 |
|---|---|---|
| Miglyol 812 | 20 % | 20 % |
| Capryol 90 | 20 % | 16 % |
| Cremophor RH 40 | 60 % | 64 % |

• The micro emulsion area was the same with and without active (concentration: 10mg/g). The droplet size was identical, around 25 nm.
The initial composition of the self microemulsifying systems with (Ic) were:

| | Ratio 3:1 | Ratio 4:1 |
|---|---|---|
| Compound Ic | 1 % | 1 % |
| Miglyol 812 | 20 % | 20 % |
| Capryol 90 | 20 % | 16 % |
| Cremophor RH 40 | 59 % | 63 % |

The formation of a microemulsion was confirmed by isotropic characterization

### Particle size

In order to confirm the formation of a microemulsion, its thermodynamic stability was verified after storage in aggressive conditions and after high dilution in water or physiological fluids.

The following samples were tested:
- S/CoS Ratio 3:1: 86 % of water, 3 % of oily phase, 8 % of Cremophor RH40 and 3 % of Capryol 90
- S/CoS Ratio 4:1: 86 % of water, 3 % of oily phase, 9 % of Cremophor RH40 and 2 % of Capryol 90
   The stability of the microemulsion was verified by the measurement of the droplet size before and after the storage in aggressive conditions and after dilution. The analysis (quasi-elastic light scattering) was performed with the equipment Coulter Nanosizer N4+.

### Storage in aggressive conditions

The samples were subjected to aggressive conditions: 2 weeks at 50°C, temperatures cycles between -15°C and +50°C for 24 hours.

The results on droplet size (expressed in nm) associated with polydispersity index obtained after storage of 2 weeks at 50°C are the following:

| | T0 | After 1 week | After 2 weeks |
|---|---|---|---|
| Ratio 3:1 | 24 (0.255) | 23 (0.255) | 25 (0.244) |
| Ratio 4:1 | 22 (0.278) | 22 (0.219) | 23 (0.233) |

The results on droplet size (expressed in nm) associated with polydispersity index obtained after temperature cycles are the following:

| | Before | After |
|---|---|---|
| Ratio 3:1 | 24 (0.255) | 23 (0.227) |
| Ratio 4:1 | 22 (0.276) | 23 (0.254) |

No variation of the droplet size was observed with the applied treatments: the structure of the microemulsion was not sensitive to the high temperature or to thermal shock.

### Final formulation with Cremophor EL

The preparation of the mixture of Miglyol 812 (liquid), Cremophor RH40 (semisolid at room temperature, solidification point 28°C) and Capryol 90 (liquid) needed to heat the mixture at 60°C to obtain a homogeneous solution. In addition, the heating of the mixture could have an impact on the chemical stability of Compound Ic.

Taking into account these two issues, the proposition was made to replace Cremophor RH 40 by Cremophor EL (from same chemical family).

Cremophor EL, polyoxyl 35 castor oil, is a liquid surfactant: No heating is needed for the manufacturing.

Two tests were performed to evaluate the interest of Cremophor EL in comparison with Cremophor RH 40: design of the pseudoternary diagram for a surfactant/co surfactant ratio of 3:1 and test of the infinite diluability.

### experimental work on the selected prototypes

### Preparation of the prototypes

### Preparation of compound (Ic) solutions (10 mg/g)

- Miglyol 812N (Condea, Batch 508)
- PEG 400 - Batch 5056
- Phosal 50PG (Aventis Nattermann, Batch 228188)
- Labrafil 1944CS (Gattefossé, Batch 15195)
- Labrasol (Gattefossé, Batch 22478)
- Gelucire 44/14 (Gattefossé, Batch 14236)
- Microemulsions (Cremophor RH40 or EL, Capryol 90, Miglyol 812N)
   The weighed drug (50 mg) was dispersed in the excipient (up to 5 g), and then maintained under mechanical stirring until dissolution. Dissolution of the drug in Phosal 50PG is a critical step (5h) due to the small difference between the concentration of the solution to obtain (10 mg/g) and the maximum solubility of Compound Ic in Phosal 50PG (11.5 mg/g).

### Preparation of the compound (Ic) semi-solid matrices (10 mg/g)

- Compound Ic
- (brevet 2001, avec Labrasol/ Labrafil)
- Vitamin E TPGS (Eastman Chemicals, Batch 90001000)

The weighed drug (50 mg) was dispersed in the melted excipient (5 g), and then maintained under mechanical stirring at 50-60°C until dissolution. The mass was poured in a suppository mould and kept refrigerated overnight. For the stability studies, the melted mass was poured into hard gelatine capsule (size 1) and kept refrigerated overnight. The gelatine shell was then removed.

### in vitro behaviour with simulated gastrointestinal fluids

### Composition of the simulated media

The following simulated media were selected for the present experiment:
• Gastric medium USP, pH 1,2
• Fasted intestinal medium, pH 6,8 (ref. Dressman et al., Pharm. Res., 1998)
• Fed intestinal medium, pH 5 (ref. Dressman et al., Pharm. Res., 1998)

**Table 2 -**

| Composition of the simulated gastro-intestinal media | | |
|---|---|---|
| **Gastric medium (G)** | | |
| Potassium chloride | 2 g | |
| Hydrogen chloride 1N | 100 ml | |
| Demineralised water | qsp 1000 ml | |

| **Fed intestinal medium (IFed)** | | For 500 ml |
|---|---|---|
| Potassium hydrogenophosphate | 0.029 M | 1.97 g |
| Sodium hydroxide | qs pH 6.8 | qs pH 6.8 |
| Sodium Taurocholate | 5 mM | 1.34 g |
| Lecithin | 1.5 mM | 0.58 g |
| Potassium chloride | 0.22 M | 8.2 g |
| With or without pancreatin | 10 g ou 0 | 5g ou 0 |
| Demineralised water | qsp 11 | qsp 500 ml |

| **Fasted intestinal medium (IFast)** | | For 500 ml |
|---|---|---|
| Acid acetic | | |
| Sodium hydroxide | qs pH 5 | qs pH 5 |
| Sodium Taurocholate | 15 mM | 4.03 g |
| Lecithin | 4 mM | 1.55 g |
| Potassium chloride | 0.19 M | 7.08 g |
| With or without pancreatin | 10 g ou 0 | 5g ou 0 |
| Demineralised water | qsp 11 | qsp 500 ml |

### Experimental conditions and results

All Compound (Ic) formulations (400 mg) were diluted 1:50 in the gastric, fasted intestinal or fed intestinal medium (20 ml), then incubated during 2 hours at 37°C under mechanical stirring (300 rpm). The drug concentration was determined by HPLC before and after filtration (0.2 or 2 µm).

### Determination of the colloidal stability and self-emulsifying properties

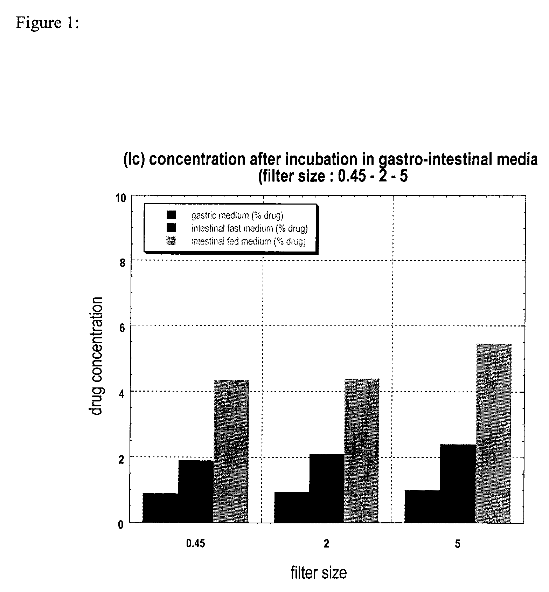

The aim of this study was to evaluate the colloidal stability and the self-emulsifying properties of the emulsion/microemulsion/micellar solution of the (Ic) formulation after incubation in the GI media. Thus, the sample was filtered onto 2 µm (able to retain oil droplets>2 µm, as well as drug crystals >2 µm) then dosed by HPLC. The filter size (2 µm) has been chosen after a screening with different filter sizes (0.45, 2 and 5 µm) tested on the aqueous solution of the drug. Indeed, as shown in Figure 1, with any filter size (0.45, 2 and 5 µm) a high retention of the drug is observed, suggesting the presence of crystals >5 µm. The filter size (in the investigated range) does not affect the retained fraction, whereas the composition of the medium drastically does. In summary, the filtered fraction was approximately 1% in the gastric medium, 2% in the fasted intestinal medium, 4.5-5.5% in the fed intestinal medium.

The data, reported in the table below and illustrated in the figure below, show that any tested formulation exhibited an improved behaviour compared to the references (Miglyol 812N and PEG400), confirming the ability of the selected excipients to self-emulsify in presence of GI fluids. The microemulsions (3:1 and 4:1), the micellar solution obtained with Vit E TPGS and the emulsion obtained with Phosal 50PG were the most homogeneous and stable systems in any medium. Nanocrystals were stable in the intestinal media, whereas a "flocculation" occurred in the gastric medium, leading to a total retention of the drug in the filter. The emulsions obtained with Labrafil/Labrasol and Gelucire 44/14 exhibited after filtration a drug concentration in the range 20-60% (Labrafil/Labrasol) and 40-90% (Gelucire 44/14). For all the novel formulations, no effect of fed conditions (pH, concentration of lecithin and biliar salts) was observed, except for Labrafil/Labrasol.

**Table 3 -**

| Compound (Ic)(µg/mL) recovered after filtration (2 µm) after previous incubation with GI media (see also Figure 1) | | | | | | | | |
|---|---|---|---|---|---|---|---|---|
| Excipients/// ///Media | Drug | PEG 400 | Gelucire 44/14 | Labrafil/ Labrasol | Migl yol | Phosal 50PG | Microemul sion Cremophor / Capryol 3/1 | Microemul sion Cremophor / Capryol 4/1 |
| Theoretical concentration | 200 µg/ml | | | | | | | |
| Gastric | 1.9 | 58.3 | 182.2 | 84.1 | 0 | 188.0 | 188.3 | 189.3 |
| Fed intestinal pH 5 | 8.8 | 20.5 | 90 | 123.6 | 2.3 | 177.0 | 187.1 | 194.2 |
| Fasted intestinal pH 6.8 | 4.2 | 14.5 | 118.4 | 39.1 | 0.6 | 175.7 | 198.8 | 195.6 |

### Conclusions on the in vitro behaviour with GI fluids

As general conclusion concerning the self-emulsifying properties and colloidal stability of the formulated drug, all the tested formulation exhibited an improved behaviour compared to the references (Miglyol 812N and PEG 400), confirming the ability of the selected excipients to self-emulsify in presence of GI fluids. The microemulsions (3:1 and 4:1), the emulsion obtained with Phosal 50PG and the micellar solution obtained with Vit E TPGS were the most homogeneous and stable systems in any medium. For all the novel formulations, no effect of fed/fasted conditions on the colloidal stability was observed, except for Labrafil/Labrasol, where the drug fraction filtered decreased from 60 to 20% in the fasted intestinal medium.

In humans, it is understood that, to choose the most appropriate daily dosage, there should be taken into account the weight of the patient, his general state of health, his age and all factors which may influence the efficacy of the treatment. Preferably, the compositions are prepared such that a unit dose contains from 0.1 to 50 mg of active product.

Among the azetidine derivatives of general formula (Ia) or (Ib), the following products are more particularly preferred:
- 1 -[bis(4-chlorophenyl)methyl]-3-[(3,5-difluoro-phenyl)(methylsulfonyl)methylene]azetidine);
- N-{1-[bis(4-chlorophenyl)methyl]azetidin-3-yl}-N-pyrid-3-ylmethylsulfonamide
- N-{1-[bis(4-chlorophenyl)methyl]azetidin-3-yl}-N-(3,5-difluorophenyl)methylsulfonamide

It is understood that the compositions according to the invention, containing these products, are particularly preferred.

In the alternative, where a second active ingredient is introduced, the compositions may comprise 0.2 to 50 mg in the case where the associated product is sibutramine. However, this quantity may optionally be lower and may vary from 0.2 to 10 mg.

In the case where the associated product is L-dopa, the compositions may comprise 100 to 300 mg of this second active ingredient, preferably 250 mg.

The stabilizing agents may be, for example, antioxidants chosen in particular from α-tocopherol, ascorbyl palmitate, BHT (butyl hydroxytoluene), BHA (butyl hydroxyanisole), propyl gallate or malic acid for example;

The preservatives may, by way of example, be chosen from sodium metabisulfite, propylene glycol, ethanol or glycerin;

Among the agents capable of adjusting the viscosity, there may be mentioned, for example, lecithins, phospholipids, propylene glycol alginate, sodium alginate or glycerin;

The agents capable of modifying the organoleptic properties of the composition are, by way of example, malic acid, fumaric acid, glycerin, vanillin or menthol.

When such additives are used, the latter may constitute from 0.001% to 5% by weight of the total composition.

According to the invention, the pharmaceutical composition may be obtained by mixing, where appropriate, the principal excipients (after heating if necessary, in the case of solid or semisolid excipients), and then, if necessary, mixing with the additional additives, followed by the addition of the azetidine derivative of general formula (Ia) or (Ib) and, where appropriate, of the active ingredient capable of potentiating the effects of the azetidine derivative of general formula (Ia) or (Ib), and maintaining stirred in order to obtain a homogeneous mixture.

The use of this process is described in greater detail below in the examples.

The compositions according to the invention may be provided in the liquid, solid or semipasty state.

They are particularly suitable for presentation in the form of hard gelatin capsules or soft gelatin capsules, or in the form of an oral solution.

The compositions according to the invention are particularly advantageous because of their good stability, both physically and chemically, and the enhancement of the bioavailablity which they offer upon oral administration of the azetidine derivatives of general formula (Ia) or (Ib).

According to another alternative of the invention, the preferred compositions as defined above, containing at least one active ingredient of general formula (Ia) or (Ib), may be administered before, simultaneously with or after the administration of an active ingredient capable of potentiating the effects of the azetidine derivative of general formula (Ia) or (Ib).

It is understood that the presentation kits comprising, on the one hand, a preferred composition according to the invention as defined above and, on the other hand, a composition comprising the active ingredient capable of potentiating the effects of the azetidine derivative of general formula (Ia) or (Ib) also fall within the scope of the present invention. It is also understood that the presentation kits may contain, as composition capable of potentiating the effects of the azetidine derivative of general formula (Ia) or (Ib), compositions comprising sibutramine, or comprising an agent that activates dopaminergic neurotransmission in the brain.

## Claims

1. A stable pharmaceutical composition comprising at least one azetidine derivative of general formula: 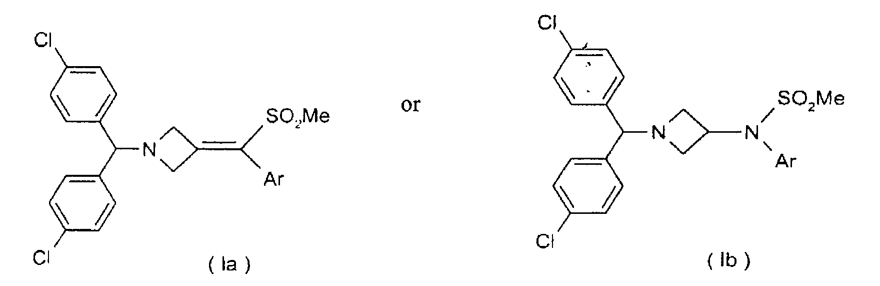 in which Ar is an aromatic or heteroaromatic group optionally substituted with one or more (C1-C4)alkyl, halogen, NO₂, CN, (C1-C4)alkoxy or OH groups, optionally in combination with another active ingredient capable of potentiating the effects of the azetidine derivative of general formula (Ia) or (Ib), in a system comprising:
(i) a phospholipid, or
(ii) a combination of Miglyol, capriol and cremophor.

2. The pharmaceutical composition as claimed in claim 1, wherein the at least one azetidine derivative is

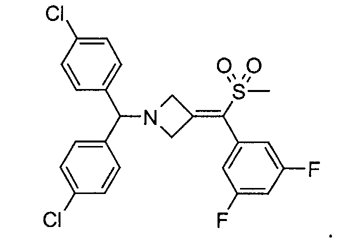

3. The pharmaceutical composition as claimed in claim 1, wherein the formulation contains up to 200 mg azetidine derivative per g.

4. The pharmaceutical composition as claimed in one of claims 1-3, which further comprises an additional additive chosen from stabilizing agents, preservatives, agents which make it possible to adjust the viscosity, or agents which can modify, for example, the organoleptic properties.

5. The pharmaceutical composition as claimed in claim 1, wherein the active ingredient derived from azetidine, is present in an amount of 0.01 to 70% by weight of the total composition.

6. A process for preparing a composition as claimed in one of claims 1 to 5, wherein there is prepared, where appropriate, the mixture of principal excipients, after heating, if necessary, in the case of the solid or semisolid excipients, and then, if necessary, the mixture with the additional additives, and then the azetidine derivative as defined in claim 1 and, where appropriate, the active ingredient capable of potentiating the effects of the azetidine derivative of general formula (Ia) or (Ib), defined in claim 1 are added and stirring is maintained in order to obtain a homogeneous mixture.

7. A presentation kit containing a composition as claimed in claim 1 or 2 and a composition comprising an active ingredient capable of potentiating the effects of the azetidine derivative defined in claim 1.

8. The presentation kit as claimed in claim 7, wherein the active ingredient capable of potentiating the effects of the azetidine derivative is sibutramine.

9. A presentation kit containing a composition as claimed in claim 1 or 2 and a composition comprising an agent that activates dopaminergic neurotransmission in the brain.
